# EUROPÄISCHE PATENTSCHRIFT

(11) **EP 1 189 857 B1**
(45) Veröffentlichungstag und Bekanntmachung des Hinweises auf die Patenterteilung: **26.05.2004**
(21) Anmeldenummer: 00935215.4
(22) Anmeldetag: 16.06.2000
(51) Int. Cl.: C07C 29/00

(54) **VERZWEIGTE, WEITGEHEND UNGESÄTTIGTE FETTALKOHOLE**
BRANCHED LARGELY INSATURED FATTY ALCOHOLS
ALCOOLS GRAS RAMIFIES ET LARGEMENT INSATURES

(30) Priorität: 25.06.1999 US 141150 P
(43) Veröffentlichungstag der Anmeldung: 27.03.2002
(73) Patentinhaber: Cognis Deutschland GmbH & Co. KG, 40589 Düsseldorf (DE)
(72) Erfinder: WESTFECHTEL, Alfred, D-40724 Hilden (DE); DOWNING, Tom, Park Hills KY 41011 (US); BLEWETT, Bill, Lakeside Park, KY 41017 (US); KENNEDY, Steve, Cincinnati, OH 45243-2343 (US); HÜBNER, Norbert, D-40597 Düsseldorf (DE); FRIESENHAGEN, Lothar, D-40589 Düsseldorf (DE); PELZER, Gerrit, D-40589 Düsseldorf (DE); KLEIN, Norbert, D-40822 Mettmann (DE); BEHLER, Ansgar, D-46240 Bottrop (DE)
(86) Internationale Anmeldenummer: PCT/EP2000/005538
(87) Internationale Veröffentlichungsnummer: WO 2001/000549

(56) Entgegenhaltungen:
- DE-A- 4 335 781
- DE-C- 4 422 858

## Beschreibung

### Gebiet der Erfindung

Die Erfindung befindet sich auf dem Gebiet der oleochemischen Grundstoffe und betrifft ungesättigte Fettalkohole, die sich infolge von Verzweigungen in der Kohlenwasserstoffkette gegenüber linearen Homologen durch signifikant verbesserte Eigenschaften auszeichnen, Verfahren zu deren Herstellung sowie ihre Verwendung zur Herstellung von oberflächenaktiven Mitteln.

### Stand der Technik

Ungesättigte Fettalkohole, die im wesentlichen auf Basis von Rindertalg durch Spaltung der Triglyceride, Umnetztrennung der Fettsäuren [Römpp, Lexikon der Chemie, Thieme Verlag, Stuttgart, S. 4811 f (1992)] in die weitgehend gesättigte Stearin- und die überwiegend ungesättigte Oleinfraktion, Veresterung des Oleins und nachfolgende Hydrierung der Methylester unter Erhalt der Doppelbindungen erhalten werden, stellen wichtige Rohstoffe für die Herstellung sowohl von kosmetischen Zubereitungen als auch Wasch-, Spül- und Reinigungsmitteln dar. So werden die Alkohole selbst beispielsweise als Betontrennmittel eingesetzt, ihre Derivate, wie z.B. die Ethoxylate, Sulfate und Ethersulfate finden Verwendung als Emulgatoren oder Tenside in Shampoos und Flüssigwaschmitteln, Oleylester dienen vielfach als kosmetische Ölkomponenten. Die vorteilhaften Eigenschaften dieser Stoffe sind an das Vorhandensein der Doppelbindung im Molekül geknüpft, was gleichzeitig jedoch auch Probleme aufwirft, da die ungesättigten Fettalkohole leicht der Autoxidation anheimfallen, was mit Verfärbungen und unerwünschten chemischen Veränderungen (z.B. Bildung von Peroxiden und Hydroperoxiden) verbunden ist.

Es ist deshalb klar, daß im Markt der Wunsch nach .ungesättigten Fettalkoholen mit verbesserter Oxidationsstabilität oder geeigneten Ersatzstoffen besteht, welche über mindestens gleichwertige anwendungstechnische Eigenschaften verfügen. Als Alternative für ungesättigte Fettalkohole haben indes bislang nur mehr oder minder reine Isostearylalkohole zur Verfügung gestanden. Zu deren Herstellung ist es indes erforderlich, zunächst Ölsäure zu dimerisieren [Fat Sci. Technol. 93, 340 (1991)], die Fraktion monomerer, verzweigter Fettsäuren abzutrennen, zu härten, einer fraktionierten Kristallisation zu unterwerfen, die dabei anfallende flüssige, isostearinsäurereiche Fraktion abzutrennen, mit Methanol zu verestern und die Ester anschließend zu den Alkoholen zu hydrieren.

Das oben geschilderte Verfahren ist indes durch die zwei Hydrierschritte technisch aufwendig und liefert mit den Isostearylalkoholen Ersatzstoffe, die die ungesättigten Fettalkohole nur bedingt ersetzen können. Somit hat die Aufgabe der vorliegenden Aufgabe darin bestanden, ungesättigte Fettalkohole zur Verfügung zu stellen, die sich bei mindestens vergleichbaren anwendungstechnischen Eigenschaften durch eine verbesserte Oxidationsstabilität auszeichnen.

### Beschreibung der Erfindung

Gegenstand der Erfindung sind verzweigte, ungesättigte Fettalkohole, dadurch erhältlich, daß man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, weitgehend ungesättigten Fettsäuren in die entsprechenden Fettsäuremethylester überführt und
(d) die verzweigten, weitgehend ungesättigten Fettsäuremethylester unter Erhalt der Doppelbindungen hydriert.

Überraschenderweise wurde gefunden, daß die verzweigten, ungesättigten Fettalkohole gegenüber den linearen Homologen gleicher Kettenlänge und gleicher lodzahl eine deutlich verbesserte Autoxidationsstabilität aufweisen.

### Herstellung der ungesättiglen Fettalkohole

Ein weiterer Gegenstand der Erfindung betrifft ein Verfahren zur Herstellung von verzweigten, ungesättigten Fettalkoholen, bei dem man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, verzweigten ungesättigten Fettsäuren in die entsprechenden Fettsäuremethylester überführt und
(d) die verzweigten, ungesättigten Fettsäuremethylester unter Erhalt der Doppelbindungen hydriert.

Die Dimerisierung von Fettsäuren und die Gewinnung von Monomerfettsäuren aus den Dimerisaten ist aus dem Stand der Technik hinreichend bekannt. In diesem Zusammenhang sei beispielsweise auf die Übersichten von A.Behr et al. **[Fat Sci.Technol. 93, 340 (1991)]** sowie H. Möhring et al. **[ibid. 94, 41 (1992) und 94, 241 (1992)]** verwiesen. Die Abfolge der Schritte (a) bis (d) liefert auf Basis von dimerisierten, vorzugsweise einfach ungesättigten C₁₆- bis C₂₂-Fettsäuren, also Ölsäure, Elaidinsäure, Petroselinsäure, Gadoleinsäure und Erucasäure sowie deren Gemischen verzweigte, weitgehend ungesättigte Fettalkohole im lodzahlbereich von 45 bis 85. Für eine Reihe von Anwendungen ist dies zweifellos völlig ausreichend, werden jedoch Fettstoffe benötigt, die einen höheren Gehalt an ungesättigten Verbindungen aufweisen, empfiehlt es sich, daß man die bei der Dimerisierung anfallende Monomerfraktion zunächst einer fraktionierten Kristallisation unterwirft und die dabei anfallende flüssige Phase gegebenenfalls nach Destillation der Veresterung unterwirft. Die dabei anfallende Fettsäure und deren Methylester stellen eine schon ziemliche reine Isoölsäure bzw. einen Isoölsäuremethylester dar, die eine lodzahl im Bereich 75 bis 95 aufweisen. In jedem Fall ist es ratsam, die Methylester und/oder die Fettalkohole einer Destillation und/oder fraktionierten Kristallisation ("Winterisierung") zu unterwerfen. Die Veresterung der Fettsäuren mit Methanol erfolgt nach den Verfahren des Stands der Technik und dient dazu Methylester zu erzeugen, die sich vergleichsweise leicht hydrieren lassen. Anstelle der Methylester können selbstverständlich auch andere Niedrigalkylester, wie z.B. Ethyl-, Propyl- oder Butylester erzeugt und dann hydriert werden, die Auswahl des Alkohols ist an sich unkritisch und richtet sich ausschließlich nach wirtschaftlichen Kriterien und Verfügbarkeit. Anstelle der Methyl- bzw. Niedrigalkytester ist es grundsätzlich auch möglich, die Fettsäuren direkt zu verestem, allerdings werden für diesen Zweck dann spezielle Katalysatoren benötigt, die mit den Säuren keine Salze bilden; zudem muß das Reaktormaterial korrosionssicher sein. Auch die Hydrierung der ungesättigten Methylester zu den entsprechenden Alkoholen kann in an sich bekannter Weise erfolgen. Entsprechende Verfahren und Katalysatoren, insbesondere solche auf Basis von Kupfer und Zink, sind beispielsweise den folgenden Druckschriften zu entnehmen: **DE 4335781 C1, EP 0602108 B1, US 3193586** und **US 3729520** (Henkel); auf den Inhalt dieser Schriften wird ausdrücklich Bezug genommen.

### Gewerbliche Anwendbarkeit

Die neuen verzweigten, ungesättigten Fettalkohole zeichnen sich durch besondere Oxidationsstabilität aus und eignen sich daher zur Herstellung von oberflächenaktiven Mitteln, vorzugsweise Wasch-, Spül-, Reinigungs- und Avivagemitteln sowie kosmetischen und/oder pharmazeutischen Zubereitungen, in denen sie in Mengen von 1 bis 50, vorzugsweise 5 bis 35 und insbesondere 10 bis 25 Gew.-% enthalten sein können.

### Beispiele

**Beispiel 1**. 23 kg Monomerfettsäure Edenor® 935 (Henkel KGaA) wurden mit 20 kg Methanol 2 h bei 240 °C und 100 bar verestert Nach Abtrennen der Wasser/Methanol-Mischung wurde die gleiche Menge Frisch-Methanol zugesetzt und der Vorgang zweimal wiederholt. Der so erhaltene Ester besaß eine Säurezahl von 0,8. Der Methylester wurde in der Festbettfahrweise an einem Zn-Cr-Katalysator unter Erhalt der Doppelbindung hydriert. Hierbei wurden pro Stunde 0,5 Volumeneinheiten Methylester - bezogen auf das Gesamtvolumen der Anlage - durchgesetzt. Nach Abtrieb des Methanols wurde der Rohalkohol destilliert (3% Vorlauf, 90 % Hauptlauf, 6% Rückstand). Der resultierende Alkohol zeigte eine Hydroxylzahl von 192, eine Verseifungszahl von 0,9 und eine lodzahl von 74; der Festpunkt betrug 25,8 °C.

**Beispiel 2.** Monomerfettsäure wurde durch Kristallisation aus Methanol/Wasser (Emersol-Verfahren) von geradkettigen, gesättigten Fettsäuren weitgehend befreit. Auf diese Weise wurden ca. 20 Gew.% Fettsäure, überwiegend Palmitin- und Stearinsäure, abgetrennt. Die nach Abdestillation des Lösemittels erhaltene flüssige Fettsäuremischung besaß einen Titer von 5 °C und wurde analog Beispiel 1 zunächst in den Methylester überführt und dann zum ungesättigten Fettalkohol hydriert. Dieser zeigte eine Hydroxylzahl von 191, eine Verseifungszahl von 1,7 und eine lodzahl von 87; der Festpunkt betrug 3,8 °C.

**Beispiel 3 und Vergleichsbeispiel V1.** Die Autoxidationsstabilität der neuen verzweigten, weitgehend ungesättigten Fettalkohole wurde gemäß der Standard-Methode AOCS CD12B92 untersucht. Der Alkohol gemäß Beispiel 2 war dabei über 90 h stabil, während ein linearer ungesättigter Fettalkohol gleicher lodzahl (HD Ocenol 80/85 V) unter gleichen Bedingungen nur eine Stabilität von 10 h aufwies.

## Patentansprüche

1. Verzweigte, ungesättigte Fettalkohole, dadurch erhältlich, daß man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, ungesättigten Fettsäuren in die entsprechenden Fettsäuremethylester überführt und
(d) die verzweigten, ungesättigten Fettsäuremethylester unter Erhalt der Doppelbindungen hydriert.

2. Verfahren zur Herstellung von verzweigten, ungesättigten Fettalkoholen, bei dem man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, ungesättigten Fettsäuren in die entsprechenden Fettsäuremethylester überführt und
(d) die verzweigten, ungesättigten Fettsäuremethylester unter Erhalt der Doppelbindungen hydriert.

3. Verfahren nach Anspruch 2, **dadurch gekennzeichnet, daß** man die bei der Dimerisierung anfallende Monomerfraktion zunächst einer fraktionierten Kristallisation unterwirft und die dabei anfallende flüssige Phase gegebenenfalls nach Destillation der Veresterung unterwirft.

4. Verfahren nach den Ansprüchen 2 und/oder 3, **dadurch gekennzeichnet, daß** man die Methylester und/oder die Fettalkohole einer Destillation und/oder fraktionierten Kristallisation unterwirft.

5. Verwendung der verzweigten, ungesättigten Fettalkohole nach Anspruch 1 zur Herstellung von Wasch-, Spül-, Reinigungs- und Avivagemitteln.

6. Verwendung der verzweigten, ungesättigten Fettalkohole nach Anspruch 1 zur Herstellung von kosmetischen und/oder pharmazeutischen Zubereitungen.

## Claims

1. Branched, unsaturated fatty alcohols obtainable by
(a) dimerizing unsaturated C₁₆₋₂₂ fatty acids in known manner,
(b) removing the monomer fraction accumulating in the dimerization step,
(c) converting the branched, unsaturated fatty acids present in this fraction into the corresponding fatty acid methyl esters and
(d) hydrogenating the branched, unsaturated fatty acid methyl esters with the double bonds intact.

2. A process for the production of branched, unsaturated fatty alcohols, **characterized in that**
(a) unsaturated C₁₆₋₂₂ fatty acids are dimerized in known manner,
(b) the monomer fraction accumulating in the dimerization step is removed,
(c) the branched, unsaturated fatty acids present in this fraction are converted into the corresponding fatty acid methyl esters and
(d) the branched, unsaturated fatty acid methyl esters are hydrogenated with the double bonds intact.

3. A process as claimed in claim 2, **characterized in that** the monomer fraction obtained in the dimerization step is first subjected to fractional crystallization and the liquid phase obtained is subjected to esterification, optionally after distillation.

4. A process as claimed in claims 2 and/or 3, **characterized in that** the methyl esters and/or the fatty alcohols are subjected to distillation and/or fractional crystallization.

5. The use of the branched, unsaturated fatty alcohols claimed in claim 1 for the production of laundry detergents, dishwashing detergents, cleaners and softeners.

6. The use of the branched unsaturated fatty alcohols claimed in claim 1 for the production of cosmetic and/or pharmaceutical preparations.

## Revendications

1. Alcools gras ramifiés, non saturés, accessibles par un procédé selon lequel
a) on dimérise des acides gras non saturés ayant de 16 à 22 atomes de carbone d'une manière connue en soi,
b) on sépare la fraction monomère qui se produit au cours de la dimérisation,
c) on transforme les acides gras ramifiés, non saturés contenus dans cette fraction en ester méthyliques d'acide gras correspondants et
d) on hydrogène les esters méthyliques d'acide gras ramifié, non saturé, tout en conservant les double liaisons.

2. Procédé de production d'alcools gras ramifiés non saturés, selon lequel
a) on dimérise des acides gras non saturés ayant de 16 à 22 atomes de carbone, d'une manière connue en soi,
b) on sépare la fraction monomère qui se produit au cours de la dimérisation,
c) on transforme les acides gras ramifiés, non saturés, contenus dans cette fraction en esters méthyliques d'acide gras correspondants et
d) on hydrogène les esters méthyliques d'acide gras ramifié, non saturés tout en conservant les double liaisons.

3. Procédé selon la revendication 2,
**caractérisé en ce qu'**
on soumet la fraction monomère qui se produit au cours de la dimérisation en premier lieu à une cristallisation fractionnée et on soumet la phase liquide qui se forme de cette façon le cas échéant après distillation, à une estérification.

4. Procédé selon les revendications 2 et/ou 3,
**caractérisé en ce qu'**
on soumet les esters méthyliques et/ou les alcools gras à une distillation et/ou à une cristallisation fractionnée.

5. Utilisation des alcools gras ramifiés, non saturés selon la revendication 1, en vue de la production de produits de lavage, de rinçage, de nettoyage et d'avivage.

6. Utilisation des alcools gras ramifiés, non saturés, selon la revendication 1, en vue de la production de préparations cosmétiques et/ou pharmaceutiques.
